Europäisches Patentamt

European Patent Office

Office européen des brevets

(11) Publication number: **0 287 210**
**A2**

# EUROPEAN PATENT APPLICATION

(21) Application number: 88302146.1

(22) Date of filing: **11.03.88**

(51) Int. Cl.⁴ **A61K 45/06 , A61K 33/30 , A61K 31/375 , A61K 31/60 , A61K 31/405 , A61K 31/52 , A61K 33/22**

(30) Priority: **13.03.87 GB 8706052**

(43) Date of publication of application:
**19.10.88 Bulletin 88/42**

(84) Designated Contracting States:
**AT BE CH DE ES FR GB GR IT LI LU NL SE**

(71) Applicant: **MEDICAL RESEARCH INTERNATIONAL LIMITED**
**2 Paddock Drive**
**Dorridge Solihull B93 8BZ(GB)**

(72) Inventor: **Skinner, Gordon Robert Bruce**
**58 Seven Star Road**
**Solihull B93 8BL(GB)**
Inventor: **Buchan, Alexander**
**278 Harborne Park Road**
**Birmingham B17 0BL(GB)**

(74) Representative: **Cuddon, George Desmond et al**
**MARKS & CLERK Alpha Tower Suffolk Street Queensway**
**Birmingham B1 1TT(GB)**

(54) **Therapeutic compositions.**

(57) A therapeutic comnposition comprises a combination of therapeutic agents whose effect is not substantially less than the sum of the separate therapeutic effects of the agents, each agent being present in an amount which will not result in an unacceptable side effect on a recipient, and the side effects of at least some of the agents are different from those of others of the agents.

EP 0 287 210 A2

Xerox Copy Centre

## "THERAPEUTIC COMPOSITIONS"

This invention relates to therapeutic compositions, and in particular embodiments to compositions which are effective against virus and bacterial infections, against cell replication and against protein synthesis in tumour explants.

With reference to antiviral compositions, since viruses are obligate intracellular parasites. effective antiviral agents must operate within the body cells to prevent or reduce virus replication. It is in most cases desirable that these agents should not be toxic to the cells as this will produce unacceptable side effects in a subject. It is a problem that a number of agents for which an antiviral effect can be demonstrated in in vitro cell systems in a laboratory have no significant therapeutic effect when used in corresponding doses in vivo in human subjects, and an increase of the dosage to levels which will produce an antiviral effect results in unwanted side effects in these subjects.

Similarly, agents against bacterial infection may have little or no effect when used singly in doses which have no undesirable side effects. Furthermore, agents which will inhibit replaciation of cells, or protein synthesis in tumours, may need to be used in doses which result in such side effects, in order to provide the required inhibition.

It is an object of this invention to provide therepeutic compositions in which the foregoing problems are overcome.

According to the invention a therapeutic composition comprises a combination of therapeutic agents each of which is present in an amount which will not result in an unacceptable side effect on a recipient and in which the side effects of at least some of said agents are different from those of others of the agents, and in which the combined therapeutic effect of said agents is not substantially less than the sum of their separate therapeutic effects.

Examples of the invention will now be described with respect to:

(i) the effect of seven therapeutic agents against herpes simplex type 1,

(ii) the effect of four therapeutic agents against staphylococcus aureus,

(iii)) the effects of four therapeutic agents against candida albicans,

(iv) the effects of four therapeutic agents on replication of oncogenic cells, and

(v) the effects of the four agents of (iii) above on protein synthesis in tumour explants.

Tables 1 to 11 relate to the effects of antiviral agents against herpes simplex type 1.

Table 1 identifies five of these antiviral agents and shows their concentrations M (millimol) used in obtaining the values shown in Tables 2 to 8.

Experimental test results R are shown in Tables 2 to 11, in which:-

R is $\log_{10}$ (virus titre in bland medium - virus titre in presence of therapeutic agent)

X is the arithmetic mean of results R

N is number of tests carried out

SE is standard error SD/ N-1, where SD is the standard deviation

C is the coefficient of variance, corresponding to 100SD/X.

The test results were obtained as follows. Monolayers of baby hamster kidney cells BHK 21 (McPherson and Stoker 1962) were prepared and infected with five plaque-forming units per cell of Type 1 Herpes Simplex Virus. The virus inoculum was removed after one hour, the cell sheets washed three times and 3 ml of fresh medium added, this fresh medium containing one or more of the therapeutic agents listed in Table 1, namely lithium succinate, zinc sulphate, ascorbic acid, acetyl salicylic acid and indomethacin, the concentrations of these components being indicated in Table 1. The cell sheets were then incubated for 16 hours following which the media were removed, the cell sheet resuspended in 1 ml of sterile water, disrupted by ultrasonic vibration and titrated for virus activity. The effect of each antiviral agent, either singly or in combination. was calculated by subtraction of the virus titre following incubation of the agent or agents. from the virus titre when the cultures were incubated in medium containing no antiviral agent.

Table 2 shows the antiviral effects of the agents and concentrations listed in Table 1, used singly and in all combinations.

It will be seen from this table that the antiviral effect of the five agents is cumulative, whether used singly or in any combination of up to five, and that the combined use of all five agents resulted in a highly significant reduction in virus titre of over 1000 fold. It is also clear that the combination of acetyl salicylic acid and indomethacin had a synergistic effect in that when used in combination they gave a significantly greater reduction in virus titre than the sum of the reduction in virus titre resulting from use of those two agents independently (Tables 3-8).

Table 3 shows test results R obtained from acetyl salicylic acid A and indomenthacin I when used

2

singly and in combination (AI). A sum (A + I) of the separate values is included for comparison with the results of the combination (AI). In Table 3 the highest values of A and I have been summed and the lowest values have also been summed to maximise the variance in A + I prior to comparison with AI.

In each of Tables 4-8 each value is derived from a series of replicate testings and is a compound mean of additional data which is available for statistical analysis. Each of Tables 4-8 show test results from acetyl salicylic acid A and indomethacin I with one or more additional antiviral agents. Table 4 shows results with one additional agent, Tables 5-7 show results with two additional agents and Table 8 results obtained with three additional agents. In Tables 4-8 where two agents are shown in conjunction this indicates that a combination of these agents was introduced to a culture medium. A plus sign indicates that test results from a single agent or combination are to be added. It will be seen, for example from Table 5 that the combination LZAI has considerably greater antiviral effect than the sum of those agents listed in that table.

Table 9 shows test results R obtained with the five agents listed in Table 1 together with a sixth agent, boric acid (B), the concentrations of each of the six agents being below that which would be expected to produce significant antiviral effect. It will be seen that the $\log_{10}$ reduction in virus titre obtained from a combination (LZCAIB) of these agents was greater than their predicted additive effect (L + Z + C + A + I + B). Table 10 shows results obtained with the above six agents used at half the concentrations shown in Table 9. In this case also the reduction in virus titre resulting from the combination was greater than the predicted additive effect.

Table 11 shows tests results R of $\log_{10}$ reduction in virus titre for lithium succinate (L) and an additional antiviral agent acycloguanosin (Zo) which is commercially available under the name Zovirax from the Wellcome Drug Company. In this table the concentrations of lithium are in millimol and the concentrations of acycloguanosin are in mg/ml.

Table 12 shows a comparison between the concentrations in plasma of the seven antiviral agents discussed above, for values D of those agents at a usual dosage level, values T which would normally be expected to result in a toxic side effect, and the levels E used in the experiemnts associated with Tables 9 to 11. It will be seen that the concentrations used in experiemnts 9 to 11 are either significantly below the toxic levels or, in the case of boric acid, at the lower end of the toxic level Values of D and T are not given for vitamin C since that substance is not detectably toxic at any level. The toxic level of acycloguanosin is shown as being greater than 0.005 mg/ml, but it is not at present clear what that level is. It will be seen from Table 11 that the levels of acycloguanosin used in tests were in every case lower than the lowest toxic level. In a significant number of the combinations shown in Table 11 the antiviral effect of the combination LZo was not substantially less than the sum of the separate agents.

It is envisaged that patients with, for example, primary or recurrent herpes genitalis may be treated by adminstration of combinations of such antiviral agents. The dosage of each agent will be such as to produce a level in the recipient's blood which, while not therapeutic on its own, will be therapeutic in combination with other agent or agents, and will therefore produce a significant therapeutic effect without side effect in the patient.

By way of example a patient would be given a combination of lithium succinate, zinc sulphate, acetyl salicylic acid, indomethacin and vitamin C, each in non-toxic doses sufficient to obtain a significant antiviral effect both in vitro and in vivo.

Tables 13 to 17 relate to the effects of antibacterial agents against staphylococcus aureus.

Table 13 identifies the four agents tested and shows their concentrations M in mMol or mass/ml as appropriate. Experimental results are shown in Tables 13 to 16 in which:

R is $\log_{10}$ (reduction in bacterial replication with agent used singly)

$R_c$ is $\log_{10}$ (reduction in bacterial replication with indicated combinations of agents)

Staphylococcus aureus, strain Bate, was used in these experiments. Drugs were added to appropriate concentrations in nutrient broth containing approximately $10^4$ micro-organisms which was then incubated for six hours at 37°C on a shaker. The replication of organisms in the presence of various combinations of drugs was compared to the replication in control nutrient broth cultures; the results have been expressed as the logarithmic reduction ($\text{Log}_{10}$) in bacterial replication in cultures in the presence of drug compared to cultures in nutrient broth controls.

The inihibitory effect of the four drugs used in dual combinations is shown Table 14. When cycloheximide, beryllium sulphate and benzyl penicillin were combined, there was a significant inhibition of replication when these drugs were used singly with the exception of anthraquinone which was used at a concentration which gave significant inhibition of replication when used singly. Therefore while there was evidence of additiveness when anthraquinone was added to beryllium sulphate or benzyl penicillin, the experiment was repeated using anthraquinone at a lower concentration, namely 0.6mg/ml (Table 15). Drug combinations without anthraquinone were excluded in this latter experiment. There was evidence of significant inhibition of bacterial replication when anthraquinone was used in combination with berullium sulphate or in combination with benzyl penicillin with no significant inhibition of bacterial replication when these three drugs were used singly. It was interesting that in both experiments there was no additive effect on bacterial replication when cycloheximide was combined with anthraquinone (Tables 14 and 15).

The combinative effect of these drugs used in triple or quadruple combination is shown in Table 16. There was evidence of additiveness with every triple and quadruple combination but as anthraquinone singly gave a significant inhibition of bacterial replication it was necessary again to re-explore these tests using anthraquinone at the reduced concentration 0.6mg/ml (Table 17). (Once again, combinations without anthraquinone were excluded). There was evidence of significant bacterial replication using the triple combination of anthraquinone, beryllium sulphate and benzyl penicillin and the quadruple combination of all four drugs while there was no significant inhibition of bacterial replication by any of these drugs used singly. Again it was interesting that the cycloheximide seemed in certain circumstances to be even inhibitory rather than additive as the dual combination of beryllium sulphate and benzyl penicillin gave a lesser effect when used with cycloheximide (Table 16) than when used in the absence of cyclohexamide (Table 14).

In summary these data provide evidence that certain drugs, which. when used singly. will not significantly inhibit bacterial replication, will significantly inhibit bacterial replication when used in dual, triple or quadruple combination.

Tables 18 and 19 relate to the effects of the same drugs as used in the preceding experiemnts, in this case against replication of candida albicans. Table 18 identifies the concentrations used for these experiments and Table 18 shows the results R and Rc for the drugs used singly and in a quadruple combination. It will be seen that there was a notable inhibition of replication of Candida albicans when all four drugs were used in combination. However, as beryllium sulphate did give a significant but smaller inhibition of replication, this experiment was repeated using lower concentrations of cycloheximide and beryllium sulphate.

Tables 20 to 22 relate to the effects of four drugs on replication of oncogenic cells, Table 20 showing the drugs and their concentrations M. Tables 21 and 22 show the experimental results R and Rc for the drugs when used in single, double, triple or quadruple combinations.

Baby hamster kidney cells, a semi-continuous line of cells were used in these experiments (Macpherson and Stoker. Virology (1962), 147-151). Monolayers of cells were prepared by seeding $5 \times 10^5$ cells in plastic Petri dishes and incubating overnight in supplemented Eagles medium containing.10% calf serum and 10% tryptose phosphate broth at 37°C in a humidified incubator. The four drugs were then added to appropriate concentrations in the medium and cell replication measured over a period of 48 hours following which the medium was removed, the cells harvested with trypsin-versene and counted in a cell counting chamber. Inhibition of cell replication was measured by calculating ($Log_{10}$) reduction in cell counts from monolayers which had been incubated with drugs compared to monolayers which had been incubated in control medium.

When these drugs were used in dual combination, a significant inhibition of cell replication was noted with most drug combinations with the eception of anthraquinone combined with 5-fluorouracil or beryllium (Table 21). When these drugs were used in triple and quadruple combination (Table 22), there was a striking inhibition of cell replication by all triple and quadruple drug combinations with the exception of the triple combination of cycloheximide, 5-fluorouracil and anthraquinone where inhibition of cell replication did not reach levels of statistical significance. In these studies there was a suggestion of synergism in every triple and quadruple combination in that the effect of these combinations was significantly higher than their expected combined effect (Table 22).

In summary there was evidence that dual, single or quadruple combinations of these drugs would significantly inhibit cell replication in situations where each drug used singly did not inhibit cell replication.

Tables 23 and 24 relate to the effects of the four drugs used in the preceding experiment in inhibiting protein synthesis in tumour explants. Table 23 showing the concentrations of these drugs. Experimental results are shown in Table 24 in which:

R is $log_{10}$ (reduction in TCA precipitable counts in response to a drug used singly)

Rc is $log_{10}$ (reduction in TCA precipitable counts in resonse to dual combinations of the drugs)

It should be noted that the first set of results for Cy and F were obtained in a separate experiment from that used to obtain the rest of the data in this table. It will also be noted that in the second set of results for Cy and F the combination was not tested.

Tumour explants made from sarcomata induced in hamsters by inoculation of BHK-21 cells (Macpherson and Stoker 1962) were established in organ culture as described by Cowan et al, British Journal of Experimental Pathology (1982) at 125-132 and 472-478. Following 24 hours in culture in varying concentrations and combinations of the drugs, approximately $25\mu$ Ci of $^{35}$S methionine were added to each explant culture which was then incubated for 72 hours. Inhibition of protein synthesis was measured by calculating the logarithm reduction ($Log_{10}$) in trichloroacetic acid (TCA) precipitable counts in the presence of the drug from the number of TCA precipitable counts in tumour explants that had been incorporated into explants in control medium.

The concentration of drugs used in these experiments is shown in Table 23. There was inhibition of protein synthesis by dual combinations of the four drugs ranging from a $log_{10}$ reduction of 0.12 for the combination of cycloheximide and anthraquinone to a $log_{10}$ reduction of 0.32 for cycloheximide and beryllium sulphate (Table 24).

Explant sample were analysed by polyacrylamide gel electrophoresis; these are shown for the drug combinations anthraquinone and cycloheximide or anthraquinone and beryllium sulphate in Figure 1. There was a decrease in the number of protein bands and in the number of synthesised protein bands when the drugs were used in combination while there was no significant difference from control explants when these drugs were used singly. There was therefore a difference in both the continuance of preformed proteins and of newly-synthesised protein when these drugs were used in dual combination.

Though the foregoing examples relate to antiviral and antibacterial agents, and to agents for inhibiting cell replication or protein synthesis, it is to be understood that combinations of other therapeutic agents, in which the concentration of each agent is below a toxic level and in which the side effects of at least some of the agents differ from the effects of others in the combination, may be expected to have a cumulative therapeutic effect. Such combinations may also be used therapuetically against conditions which are not or are not necessarily attributable to infection.

| | | M (millimol) |
|---|---|---|
| lithium succinate | L | 1.5 |
| zinc sulphate | Z | 0.1 |
| ascorbic acid (Vitamin C) | C | 2.5 |
| acetyl salicylic acid | A | 1.0 |
| indomethacin | I | 0.2 |

TABLE 1

|  | X | S E | C | N |  |  | X | S E | C | N |
|---|---|---|---|---|---|---|---|---|---|---|
| L | .39 | .05 | 42 | 12 | | LXC | .73 | | | 2 |
| Z | .26 | .05 | 66 | 11 | | LZA | .81 | | | 3 |
| C | .13 | .04 | 96 | 10 | | LZI | 2.04 | | | 2 |
| A | .20 | .05 | 75 | 10 | | LCA | .65 | | | 3 |
| I | 1.46 | .08 | 16 | 10 | | LCI | 1.95 | | | 2 |
| LZ | .61 | .05 | 17 | 6 | | LAI | 2.39 | | | 3 |
| LC | .53 | .07 | 30 | 6 | | ZCA | .44 | | | 3 |
| LA | .63 | .07 | 25 | 6 | | ZCI | 1.9 | | | 3 |
| LI | 1.75 | .13 | 17 | 6 | | ZAI | 2.3 | | | 3 |
| ZC | .28 | .08 | 64 | 5 | | CAI | 2.4 | | | 3 |
| ZA | .49 | .04 | 16 | 6 | | LZCA | .87 | | | 1 |
| ZI | 1.65 | .08 | 10 | 6 | | LZCI | 1.82 | | | 1 |
| CA | .34 | .03 | 17 | 4 | | ZCAI | 2.47 | | | 1 |
| CI | 1.66 | .12 | 14 | 5 | | LCAI | 2.37 | | | 1 |
| AI | 2.11 | .09 | 10 | 6 | | LZCAI | 3.05 | .49 | 23 | 4 |

TABLE 2

| | A | I | A + I | A I |
|---|---|---|---|---|
| R | 0.46 | 1.81 | 2.27 | 2.50 |
| | 0.28 | 1.73 | 2.01 | 2.16 |
| | 0.35 | 1.67 | 2.02 | 2.06 |
| | 0.22 | 1.54 | 1.76 | 2.07 |
| | 0.21 | 1.50 | 1.71 | 2.01 |
| | 0.20 | 1.48 | 1.68 | 1.85 |
| | 0.18 | 1.29 | 1.47 | |
| | 0.08 | 1.24 | 1.32 | |
| | 0.04 | 1.27 | 1.31 | |
| | 0.00 | 1.03 | 1.03 | |
| x | | | 1.66 | 2.11 |
| SE | | | .13 | .09 |
| C | | | 23% | 10.3% |
| N | | | 10 | 6 |

TABLE 3

|  | R | X |
|---|---|---|
| C + A + I | 1.79 | |
| C A + I | 1.80 | 1.82 |
| C I + A | 1.86 | |
| Z + A + I | 1.92 | |
| Z A + I | 1.95 | 1.91 |
| Z I + A | 1.85 | |
| L + A + I | 2.05 | |
| L A + I | 2.09 | 2.03 |
| L I + A | 1.95 | |
| C + A I | 2.45 | 2.43 |
| C A I | 2.42 | |
| Z + A I | 2.37 | 2.45 |
| Z A I | 2.32 | |
| L + A I | 2.50 | 2.44 |
| L A I | 2.39 | |

TABLE 4

|  | R | x | SE | C | N |
|---|---|---|---|---|---|
| L + Z + A + I | 2.32 | | | | |
| L A + Z + I | 2.35 | | | | |
| L I + Z + A | 2.21 | | | | |
| L I + Z A | 2.24 | | | | |
| Z A + L + I | 2.34 | | | | |
| Z I + L + A | 2.24 | 2.23 | .04 | 5.4% | 10 |
| L Z A + I | 2.27 | | | | |
| L Z I + A | 1.93 | | | | |
| L Z + A + I | 2.27 | | | | |
| L A + Z I | 2.09 | | | | |
| L Z + A I | 2.77 | | | | |
| A I + L + Z | 2.76 | | | | |
| L A I + Z | 2.65 | 2.78 | .07 | 5.2% | 5 |
| Z A I + L | 2.71 | | | | |
| L Z A I | 3.03 | | | | |

TABLE 5

| | R | x̄ | SE | C | n |
|---|---|---|---|---|---|
| L + C + A + I | 2.37 | | | | |
| L C + A + I | 2.19 | | | | |
| L A + C + I | 2.22 | | | | |
| L A + C I | 2.29 | | | | |
| C A + L + I | 2.19 | 2.17 | .04 | 5.7% | 10 |
| C A + L I | 2.09 | | | | |
| L I + A + C | 2.08 | | | | |
| C I + A + L | 2.25 | | | | |
| L C A + I | 2.11 | | | | |
| C I + A | 1.93 | | | | |
| L C + A I | 2.63 | | | | |
| A I + L + C | 2.62 | | | | |
| C A I + L | 2.81 | 2.59 | .08 | 6.2% | 5 |
| L C A I | 2.37 | | | | |
| L A I + C | 2.52 | | | | |

TABLE 6

|  | R | x | SE | C | N |
|---|---|---|---|---|---|
| Z + C + A + I | 2.05 | | | | |
| Z C + A + I | 1.94 | | | | |
| Z A + C I | 2.15 | | | | |
| Z A + C + I | 2.08 | | | | |
| Z I + A + C | 1.98 | 2.04 | .028 | 3.9% | 10 |
| Z I + A C | 1.99 | | | | |
| C A + Z + I | 2.06 | | | | |
| Z C A + I | 1.90 | | | | |
| Z C I + A | 2.10 | | | | |
| C I + A + Z | 2.12 | | | | |
| Z C + A I | 2.39 | | | | |
| A I + Z + C | 2.49 | | | | |
| Z A I + C | 2.45 | 2.49 | .05 | 4.3% | 5 |
| C A I + Z | 2.68 | | | | |
| Z C A I | 2.47 | | | | |

TABLE 7

|  | R | x | SE | C | N | x | SE | C | N |
|---|---|---|---|---|---|---|---|---|---|
| A + L I Z C | 2.02 | 2.18 |  |  |  |  |  |  |  |
| I + L Z A C | 2.33 |  |  |  |  |  |  |  |  |
| L A + Z C I | 2.53 |  |  |  |  | 2.31 | .06 | 6.8% | 8 |
| L I + Z C A | 2.19 |  |  |  |  |  |  |  |  |
| Z A + L C I | 2.44 | 2.35 | .07 | 6.3% | 6 |  |  |  |  |
| Z I + L C A | 2.16 |  |  |  |  |  |  |  |  |
| C A + L Z I | 2.35 |  |  |  |  |  |  |  |  |
| C I + L Z A | 2.47 |  |  |  |  |  |  |  |  |
| L Z + C A I | 3.03 |  |  |  |  |  |  |  |  |
| L C + Z A I | 2.85 |  |  |  |  |  |  |  |  |
| Z C + L A I | 2.67 | 2.89 | .07 | 5.4% | 5 |  |  |  |  |
| A I + L Z C | 2.84 |  |  |  |  |  |  |  |  |
| L Z C A I | 3.06 |  |  |  |  | 2.89 | .07 | 6.4% | 8 |
| L + Z C A I | 2.86 | 2.88 |  |  |  |  |  |  |  |
| Z + L C A I | 2.63 |  |  |  |  |  |  |  |  |
| C + L Z A I | 3.16 |  |  |  |  |  |  |  |  |

TABLE 8

| | M | R | L + Zo | LZo |
|---|---|---|---|---|
| L<br>Zo | 3<br>0.0004 | 0.25<br>0.96 | 1.24 | 1.05 |
| L<br>Zo | 3<br>0.0002 | 0.25<br>0.95 | 1.20 | 0.86 |
| L<br>Zo | 3<br>0.0001 | 0.25<br>0.40 | 0.65 | 0.68 |
| L<br>Zo | 1.5<br>0.0004 | 0.15<br>0.96 | 1.11 | 1.00 |
| L<br>Zo | 1.5<br>0.0002 | 0.15<br>0.95 | 1.00 | 0.70 |
| L<br>Zo | 1.5<br>0.0001 | 0.15<br>0.40 | 0.55 | 0.42 |
| L<br>Zo | 0.75<br>0.0004 | 0.07<br>0.96 | 1.03 | 1.21 |
| L<br>Zo | 0.75<br>0.0002 | 0.07<br>0.95 | 1.02 | 0.65 |
| L<br>Zo | 0.75<br>0.0001 | 0.07<br>0.40 | 0.47 | 0.45 |

TABLE 11

|   | M (millimol) | R |
|---|---|---|
| L | 1 | 0.0 |
| Z | 0.02 | 0.14 |
| C | 5 | 0.13 |
| A | 1 | 0.19 |
| I | 0.02 | 0.09 |
| B | 2 | 0.31 |

| | |
|---|---|
| L Z V A I B | 0.92 |
| L + Z + V + A + I + B | 0.88 |

TABLE 9

|   | M (millimol) | R |
|---|---|---|
| L | 0.5 | 0.0 |
| Z | 0.01 | 0.12 |
| C | 2.5 | 0.17 |
| A | 0.5 | 0.06 |
| I | 0.01 | 0.0 |
| B | 1 | 0.05 |

| | |
|---|---|
| L Z V A I B | 0.46 |
| L + Z + V + A + I + B | 0.40 |

TABLE 10

|     | D            |        | T       | E        |
| --- | ------------ | ------ | ------- | -------- |
| L   | 0.6-1.2      | mm     | 1.5     | 0.5      |
| Z   | 0.02         | mm     | 0.4     | 0.01     |
| C   | —            |        | —       | 2.5      |
| A   | 0.15-0.7     | mm     | 3       | 0.5      |
| I   | 0.0006       | mm     | 0.02    | 0.01     |
| B   | 0.003        | mm     | 0.7-3   | 1        |
| Zo  | 0.002        | mgm/ml | > 0.005 | Table 11 |

TABLE 12

|                          |     | M           |
|--------------------------|-----|-------------|
| Cycloheximide            | Cy  | 0.15 µg/ml  |
| Anthraquinone -2 sulphonic acid | An  | 1.2 mg/ml   |
| Beryllium Sulphate       | By  | 2mM         |
| Benzyl Penicillin        | . P | 0.006 µg/ml |

TABLE 13

|          | R            | Rc   |
|----------|--------------|------|
| Cy<br>An | 0.0<br>0.18  | 0.16 |
| Cy<br>By | 0.0<br>0.0   | 0.10 |
| Cy<br>P  | 0.0<br>0.0   | 0.10 |
| An<br>BY | 0.18<br>0.0  | 0.28 |
| An<br>P  | 0.18<br>0.0  | 0.35 |
| By<br>P  | 0.0<br>0.0   | 0.44 |

TABLE 14

|  | R | Rc |
|---|---|---|
| Cy<br>An | 0.1<br>0.0 | 0.0 |
| An<br>By | 0.0<br>0.1 | 0.21 |
| An<br>P | 0.0<br>0.1 | 0.25 |

TABLE 15

|  | R | Rc |
|---|---|---|
| Cy<br>An<br>By | 0.0<br>0.18<br>0.0 | 0.29 |
| Cy<br>An<br>P | 0.0<br>0.18<br>0.0 | 0.22 |
| Cy<br>By<br>P | 0.0<br>0.0<br>0.0 | 0.10 |
| An<br>By<br>P | 0.18<br>0.0<br>0.0 | 0.44 |
| Cy<br>An<br>By<br>P | 0.0<br>0.18<br>0.0<br>0.0 | 0.38 |

TABLE 16

|     | R   | Rc   |
| --- | --- | ---- |
| Cy<br>An<br>By | 0.1<br>0.0<br>0.1 | 0.07 |
| Cy<br>An<br>P | 0.1<br>0.0<br>0.1 | 0.02 |
| An<br>By<br>P | 0.0<br>0.1<br>0.1 | 0.48 |
| Cy<br>An<br>By<br>P | 0.1<br>0.0<br>0.1<br>0.1 | 0.25 |

TABLE 17

|                              |     | M          |
| ---------------------------- | --- | ---------- |
| Cyclohex imide               | Cy  | 1.5µg/ml   |
| Anthraquinone<br>2 - sulphonic | An  | 2.5mg/ml   |
| Beryllium Sulphate           | By  | 20mM       |
| Benzyl Penicillin            | P   | 0.06µg/ml  |

TABLE 18

|  | R | Rc |
|---|---|---|
| Cy | 0.24 | |
| An | 0.03 | |
| By | 0.62 | |
| P | 0.02 | 2.50 |

TABLE 19

| | | M |
|---|---|---|
| Cycloheximide | Cy | $0.05\mu g/ml$ |
| 5 - Fluorouracil | F | $0.5\mu g/ml$ |
| Anthraquinone - 2 sulphonic acid | An | $0.3mg/ml$ |
| Beryllium Sulphate | By | 2.0mM |

TABLE 20

| | R | Rc |
|---|---|---|
| Cy<br>F | 0.12<br>0.02 | 0.26 |
| Cy<br>An | 0.12<br>0.02 | 0.21 |
| Cy<br>By | 0.12<br>0.14 | 0.29 |
| F<br>An | 0.02<br>0.02 | 0.12 |
| F<br>By | 0.02<br>0.14 | 0.23 |
| An<br>By | 0.02<br>0.14 | 0.06 |

TABLE 21

19

|  | R | Rc |
|---|---|---|
| Cy<br>F<br>An | 0.12<br>0.02<br>0.02 | 0.18 |
| Cy<br>F<br>By | 0.12<br>0.02<br>0.14 | 0.43 |
| Cy<br>An<br>By | 0.12<br>0.02<br>0.14 | 0.38 |
| F<br>An<br>By | 0.02<br>0.02<br>0.14 | 0.31 |
| Cy<br>F<br>An<br>By | 0.12<br>0.02<br>0.02<br>0.14 | 0.63 |

TABLE 22

| | | M |
|---|---|---|
| Cyclohexίmide | Cy | $0.25\mu g/ml$ |
| 5-Fluorouracil | F | $7.5\mu g/ml$ |
| Anthraquinone<br>2-sulphonic acid | An | $0.2mg/ml$ |
| Beryllium sulphate | By | $2.0mM$ |

TABLE 23

| | R | Rc |
|---|---|---|
| Cy<br>F | 0.00<br>0.14 | .41 |
| Cy<br>F | 0.0<br>0.0 | N.T. |
| Cy<br>An | 0.0<br>0.0 | .12 |
| Cy<br>By | 0.0<br>0.0 | .32 |
| F<br>An | 0.0<br>0.0 | .17 |
| F<br>By | 0.0<br>0.0 | .21 |
| An<br>By | 0.0<br>0.0 | .23 |

TABLE 24

## Claims

1. A therapeutic composition comprising a combination of therapeutic agents each of which is present in an amount which will not result in an unacceptable side effect on a recipient and in which the side effects of at least some of the agents are different from those of others of the agents, and in which the therapeutic effect of the combination is not substantially less than the sum of the separate therapeutic effects of the agents.

2. A composition as claimed in Claim 1 in which each said agent is an antiviral agent.

3. A composition as claimed in Claim 2 in which each said agent is effective against herpes simplex virus.

4. A composition as claimed in Claim 3 in which said agents are selected from the group consisting of lithium succinate, zinc sulphate, ascorbic acid, acetyl salicylic acid, indomethacin, acycloguanosine and boric acid.

5. A composition as claimed in Claim 4 in which said agents are lithium, zinc sulphate, acetylsalicylic acid and indomethacin.

6. A composition as claimed in Claim 5 which additionally comprises ascorbic acid and boric acid.

7. A composition as claimed in Claim 1 in which each said agent is an anibacterial agent.

8. A composition as claimed in Claim 1 in which each said agent inhibits replication of cells.

9. A composition as claimed in Claim 1 in which said agents in combination inhibit cell protein synthesis.

10. A compositon as claimed in Claim 1 in which at least one of said agents has no detectable therapeutic effect and in which the combination of said one agent with one or more other agents has a therapeutic effect greater than that of the sum of the separate therapeutic effects of said other agent, or agents.